# EUROPEAN PATENT SPECIFICATION

(11) **EP 4 630 020 B1**
(45) Date of publication and mention of the grant of the patent: **12.08.2026**
(21) Application number: 25705575.6
(22) Date of filing: 18.02.2025
(51) Int. Cl.: A61K 35/37, A61K 35/74, A61P 1/00, C12N 1/20

(54) **METHOD FOR FECAL MICROBIOME PURIFICATION AND USES THEREOF**
VERFAHREN ZUR FÄKALIENMIKROBIOMREINIGUNG UND VERWENDUNGEN DAVON
PROCÉDÉ DE PURIFICATION DE MICROBIOME FÉCAL ET SES UTILISATIONS

(30) Priority: 22.02.2024 EP 24382186
(43) Date of publication of application: 15.10.2025
(73) Proprietor: Microviable Therapeutics SL, 33203 Gijón (ES)
(72) Inventor: MARTÍNEZ ÁLVAREZ, Noelia, 33204 Gijón Asturias (ES); HIDALGO CANTABRANA, Claudio, 33429 La Fresneda, Siero Asturias (ES); MANRIQUE RONQUILLO, María Del Pilar, 33006 Oviedo Asturias (ES); BARRIENTOS SOTO, Desirée, 33401 Avilés Asturias (ES)
(74) Representative: Clarke, Modet y Cía., S.L.
(86) International application number: PCT/EP2025/054325
(87) International publication number: WO 2025/141228

(56) References cited:
- WO-A1-2017/103550
- HEVIA ARANCHA ET AL: "Application of density gradient for the isolation of the fecal microbial stool component and the potential use thereof", vol. 5, no. 1, 19 November 2015 (2015-11-19), XP093084206, Retrieved from the Internet <URL:https://www.nature.com/articles/srep16807.pdf> DOI: 10.1038/srep16807

## Description

### FIELD OF THE INVENTION

The present invention belongs to the field of medicine. Specifically, to the field of medical microbiology. The document discloses purified microbiome obtained from human intestinal microbiota suitable for medical treatments, specially, in the prevention and treatment of recurrent urinary infections and as an adjuvant in medical treatments that involve a disruption of the intestinal microbiota.

### BACKGROUND OF THE INVENTION

Intestinal microbiota (IM) is the set of microorganisms that reside in the intestinal tract. It is essential for protection against pathogens, modulation of the immune system and synthesis of essential compounds such as amino acids or vitamins or neurotransmitters.

In recent years, scientific evidence on the health implications of IM and its therapeutic use has increased considerably.

Fecal microbiota transplantation (FMT) has been approved as a treatment for recurrent infections of the multi-antibiotic resistant (MDRO) pathogen *Clostridium difficile,* with a demonstrated efficacy of more than 90% in numerous clinical studies (Sorbara et al., 2022. Nature Reviews Microbiology 20(6):365-80). This procedure consists of IM transplantation through the administration of fecal content from a healthy donor to the patient by colonoscopy, by oral intubation or through capsules. Its effectiveness is associated with the colonization of the donor's microbiota and consequent displacement of the pathogen. The use of FMT reverses the infection and reduces the number of reinfections, significantly improving the quality of life of patients.

Currently, the effectiveness of FMT in the treatment of infections caused by other MDR pathogens is being tested (Sorbara *et al.,* 2022). Despite its effectiveness, since it is fecal matter, it presents serious health risks, and it is necessary to develop alternative solutions that allow the use of IM devoid of fecal matter.

Urinary tract infections (UTI) constitute the second most common group of community-acquired infections and are a frequent cause of consultations in primary care. Globally, UTls account for around 150 million infections per year and 40% of hospital-acquired infections (Kenneally et al., 2022. Microbiological Research 259:127010).

Antibiotic therapy is the first-line treatment and approximately 80% of urinary pathogens show resistance to at least two antibiotics. Furthermore, antibiotics often cause short- and long-term alterations in the microbiota, adverse effects, recurrences, and the development of MDR pathogens associated with prolonged episodes of recurrent infection (rUTI). Therefore, it is imperative to find an alternative treatment to treat or prevent UTIs (Amdekar et al., 2011 Current Microbiology 63(5):484).

Approximately 30% of people who suffer from UTI develop rUTI within 6 months of initial infection (Kenneally *et al.,* 2022). Most of the pathogens that cause UTI are present in the IM, so a mechanism that has been proposed for recurrent infections is reinfection from pathogen reservoirs in the gastrointestinal tract or in the urogenital epithelial cells (Worby et al., 2022. Journal of Clinical Investigation 132(5):e158497).

Commensal IM contributes to reducing colonization by various pathogens through competition for nutrients and adhesion sites, as well as through the production of antimicrobial metabolites, changes in the pH of the medium, and suppression of pathogen virulence factors (Sturov et al., 2022. Turkish Journal of Urology 48(6):406-14).

Several studies have demonstrated a correlation between the composition and diversity of the IM with urinary tract infections and confirm that intestinal dysbiosis is a factor that predisposes to a greater development of UTIs. Furthermore, it has been shown that the presence of certain bacterial species can reduce the risk and severity of episodes (Sturov 2022).

Retrospective studies and recent cases confirm that FMT is associated with lower recurrence of UTIs (Innes et al., 2021. Frontiers in Cellular and Infection Microbiology 11), which is why clinical trials are being conducted to evaluate the administration of FMT for the prevention of UTIs. Study NCT03050515, a phase I trial, included women with recurrent UTI treated with FMT via enema. Finally, study NCT06050148 is a phase 2/3 trial investigating FMT compared to placebo in the prevention of UTIs caused by *E. coli* in patients with a history of rUTI.

In addition to its essential function in protection against pathogens, IM plays a very important role in the metabolization of xenobiotic compounds and in the regulation of the immune system (Clemente et al., 2012. Cell 148 (6): 1258-70). There is increasing evidence that demonstrates the influence of IM on the development and progression of certain tumors, and the profound effect it can have on modulating the efficacy and toxicity of various oncological treatments (Jain et al., 2021. Frontiers in Immunology 12 (February): 622064).

The influence of the microbiota on the response to immunotherapy was first demonstrated in murine model trials, in which the ability of IM to stimulate the effectiveness of certain treatments, and the attenuation of such effectiveness by antibiotic treatment was seen (Lu et al., 2022. Journal of Hematology & Oncology 15 (1): 47). Subsequently, using murine models and human studies, it was shown that the IM of people who do not respond to immunotherapy (non-responders) is different from the IM of those who do respond to treatment (responders), and has a lower immunoregulatory capacity. These results have encouraged several clinical trials in which the efficacy of FMT as an adjuvant for anti-PD-1 immunotherapy has been evaluated (Routy et al., 2023. Nature Medicine 29 (8): 2121-32). The results published so far have confirmed important differences between the IM of responders and non-responders and have shown that FMT is a safe and promising therapy to boost the efficacy of these treatments (Baruch et al. 2021. Science (New York, N.Y) 371 (6529): 602-9). Furthermore, the absence of toxicity against treatments such as immune checkpoint inhibitors has been associated with the composition of the IM (Chrysostomou et al. 2023. Gastroenterology 164 (2): 198-213). These results suggest that it is necessary to develop personalized treatments that can modify patients' IM before starting treatment.

Techniques for separating fecal microbiota from stool samples have not been deeply investigated. The proper isolation of the microbiota will lead to a product suitable for intestinal microbiota transplant or treatment of infections, and as an adjuvant in medical treatments that involve a disruption of the intestinal microbiota.

There is therefore a need to develop effective methods of fecal microbiota isolation.

Hevia *et al.,* 2015 (Hevia A et al., 2015. Sci Rep 5, 16807) discloses a procedure with a density gradient that does not affect the original microbiota composition in terms of viability, distribution and proportions. However, this process should be improved in order to obtain purified microbiota as similar as possible to the original subject's microbiome, to guarantee the best approach for a future FMT and suitability in medical treatments.

WO2016/170285 describes a fecal microbiota purification method for use in FMT which consists of a purification of the microbiota and subsequent elimination of fecal matter through sequential filtration through membranes of up to 0.5 mm. Due to the size of the filters used, a large portion of the bacterial component of the microbiota is lost.

WO 2017/103550 A1 describes a method for purifying fecal microbiota using density gradients and lyophilization and any polyol, di-pentasaccharides, maltodextrins and any mixture thereof as cryoprotectant. However, stability of the product is not proper.

An alternative isolation method and product derived therefrom is therefore an important advance in a breakthrough medical technology.

### DESCRIPTION

### BRIEF DESCRIPTION OF THE INVENTION

The invention is defined in the appended claims.

In a first aspect, the disclosure is directed to a process to obtain intestinal microbiota (IM) from a stool sample comprising the following steps:
a) homogenization of a fecal sample in saline solution with a reducing agent;
b) purification by density gradient;
c) washing with a reducing solution;
d) concentration; and
e) cryopreservation, dehydration, formulation and storage of the IM.

In a second aspect, the disclosure is directed to the intestinal microbiota (IM) obtained from the process of the first embodiment described above.

In a third aspect it is disclosed a pharmaceutical composition comprising the intestinal microbiota (IM) of the second aspect described above and a pharmaceutically acceptable ingredient.

In a fourth aspect it is disclosed the IM of the second aspect or the pharmaceutical composition of the third aspect described above, for use as a medicament.

In a fifth aspect it is disclosed the IM of the second aspect or the pharmaceutical composition of the third aspect described above, for use in the treatment of a disease which courses with alteration of the IM or for use in the prevention of secondary effects after a medical treatment, or for use in reduction of the toxicity of a medical treatment in a subject.

### BRIEF DESCRIPTION OF THE FIGURES

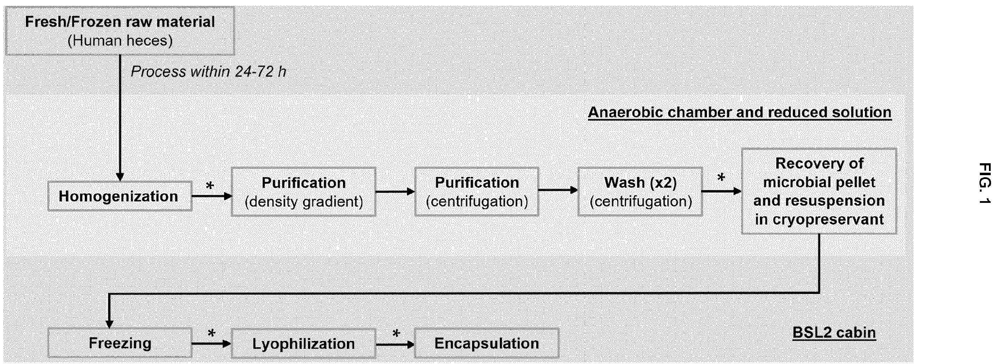

**Figure 1****. Overview of the production process.** (*) is referred to quantification and quality control. Culture-dependent methods carried out under anaerobic conditions with pre-reduced culture media.

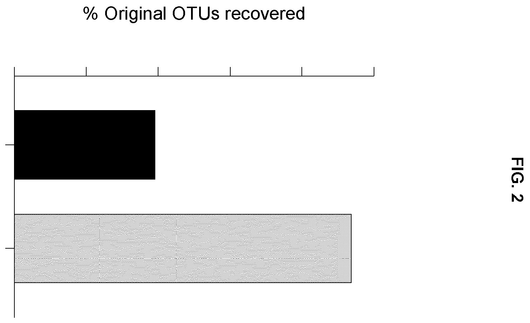

**Figure 2****. Purified microbiota with the protocol of the invention maintains a higher percentage of the bacterial OTUs found in the original fecal microbiota.** Percentage of OTUs recovered after purification process. Black: Hevia (prior art), grey: MVT-201 (purified microbiota).

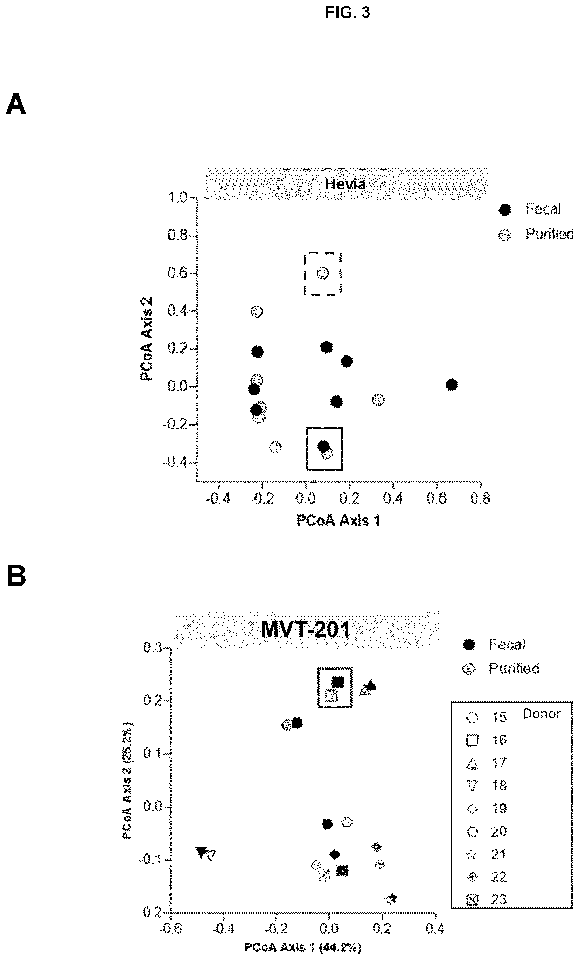

**Figure 3****. Purified microbiota with the protocol of the invention is more similar to the original fecal microbiota at the whole ecosystem level. (A)** Principal Coordinate Analysis (PCoA) comparing the overall composition of the fecal microbiota (black circles) with the purified microbiota (grey circles) using Hevia *et al.* protocol shows that, even though fecal and purified samples are overall similar (not clear separation between black and grey dots), there is not a specific pairing between fecal and purified samples. **(B)** PCoA analysis comparing the overall composition of the fecal microbiota (black) with the purified microbiota (grey) using the protocol of the invention (MVT-201), shows that each purified microbiota groups with its corresponding original microbiota (each symbol is an individual fecal donation from a different donor).

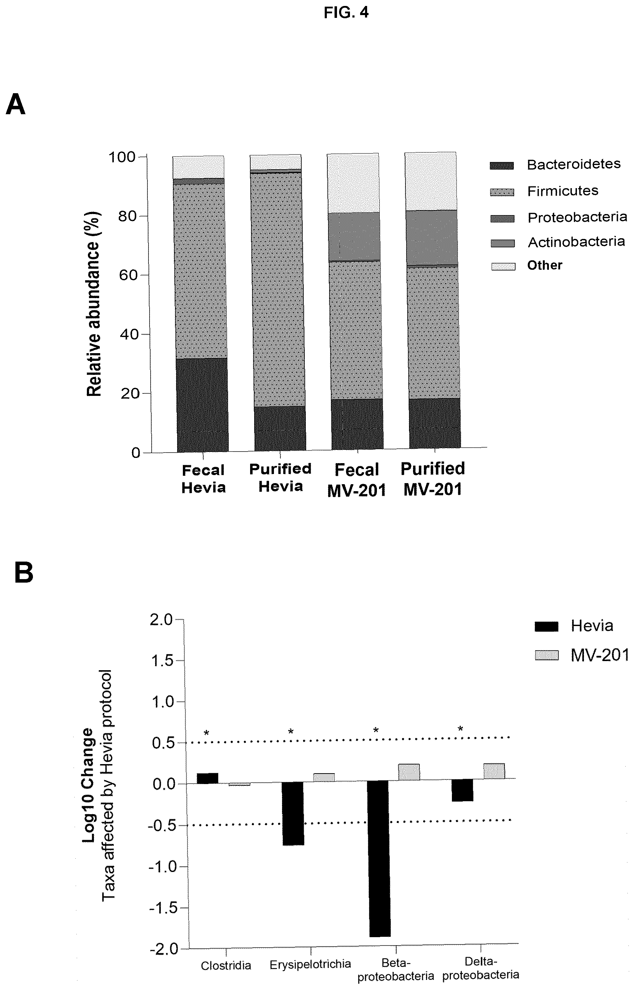

**Figure 4****. Purified microbiota with the protocol of the invention is more similar to the original fecal microbiota at specific taxa level (A)** Relative abundance of main phyla shows that the composition of the purified microbiota with prior art protocol is different from the original microbiota and that these changes are not seen using the protocol of the invention. **(B)** Changes in taxa affected by the prior art protocol showing an increase in Clostridia class and a large reduction in other taxa.

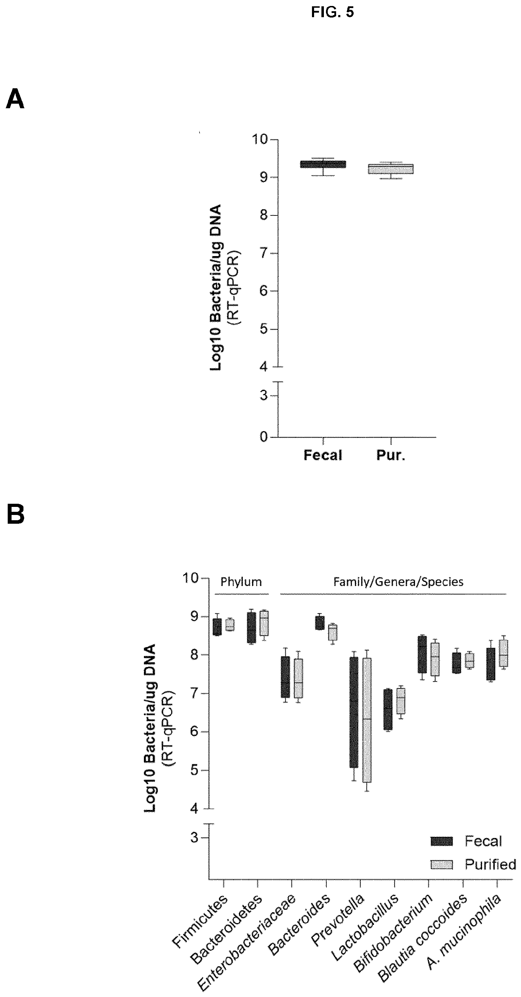

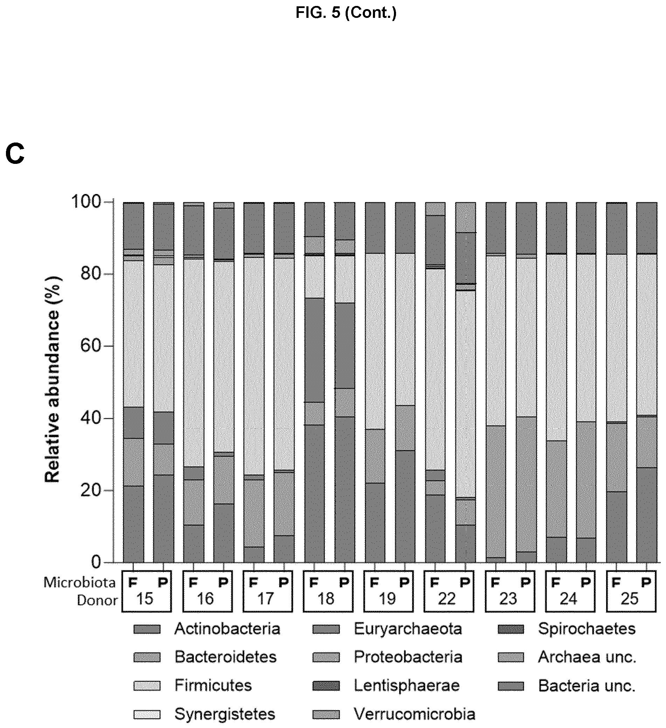

**Figure 5****. The composition contains a purified microbiota with high viability and similarity to original microbiota. (A)** Abundance of total viable bacteria in fecal microbiota sample (Fecal) or Purified microbiota (Pur.) shows no decrease in viability after purification process. **(B)** Abundance of bacteria divided by taxa in fecal microbiota sample (Fecal) or Purified microbiota (Pur.) shows no difference in viable taxa. **(C)** Relative abundance at the phylum level in the fecal microbiota (F) or Purified microbiota (P) in IM product.

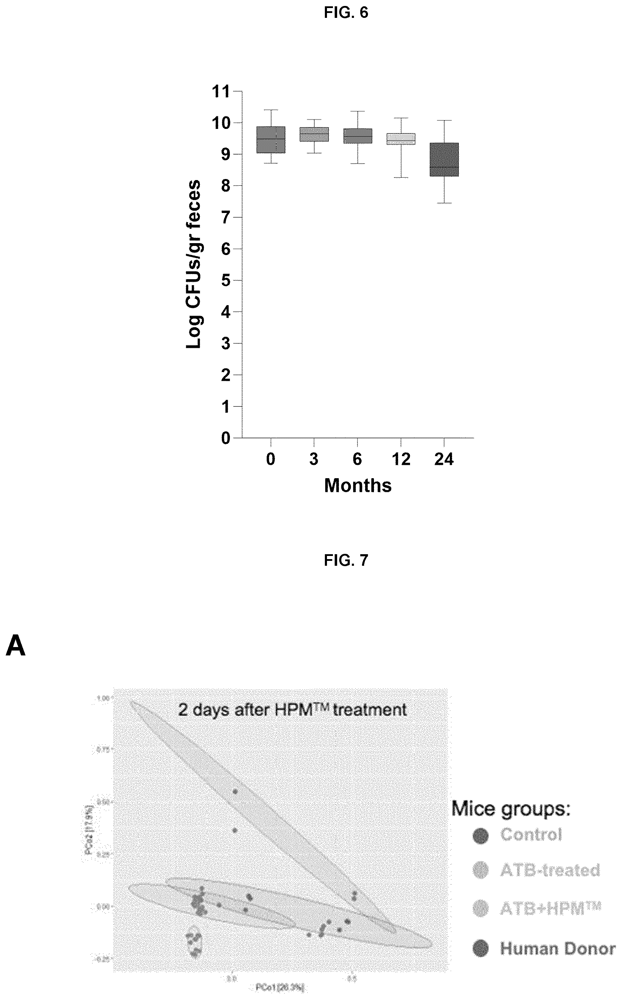

**Figure 6****. Stability of purified microbiota through time.** Bacterial quantification shows the viability of the purified microbiota is maintained for up to 12 months (maximum tested).

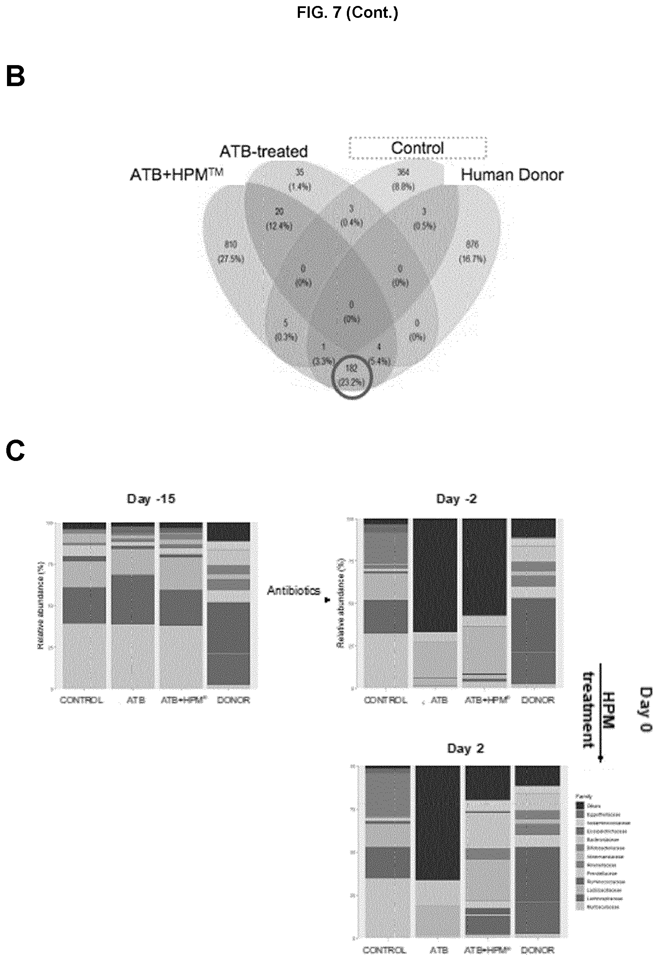

**Figure 7****. MVT-201 is safe and gets established in the intestinal tract in a murine model. (A)** Principal Coordinate Analysis (PCoA) showing the establishment of purified IM (MVT-201) within the mice intestinal tract. **(B)** Bacterial OTUs shared between control and treatment groups showing that >20% of the OTUs found in MVT-201 get established in the mice intestinal tract. **(C)** Changes in the gut microbial composition throughout the study showing that administration of MVT-201 changes the intestinal microbiota to be more similar to the donor's microbiota.

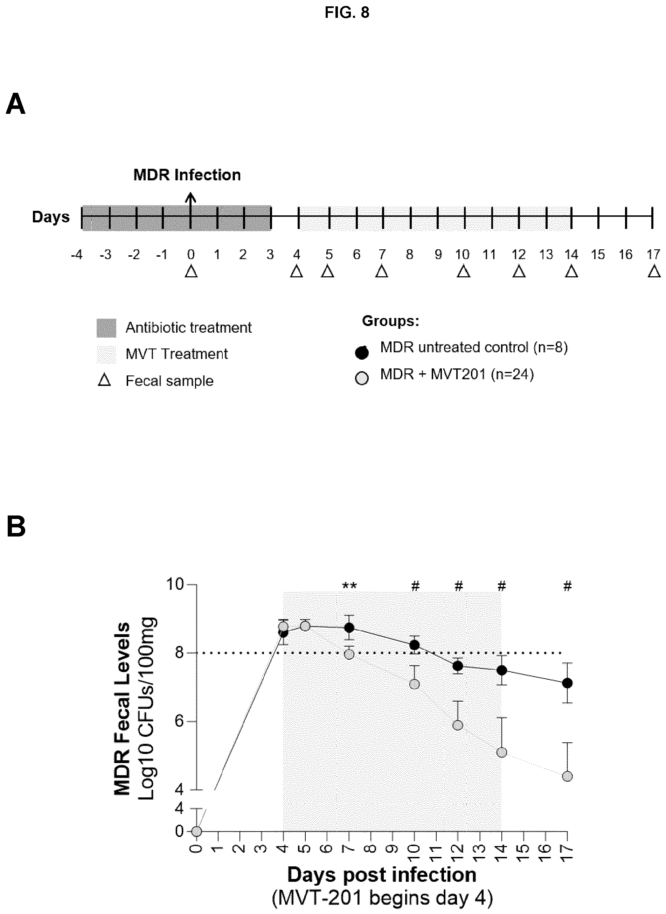

**Figure 8****. MVT-201 accelerates the reduction of a MDR intestinal pathogen. (A)** Experimental design of preclinical assay using a multi-drug-resistant pathogen (MDR) infection. **(B)** MDR pathogen levels in fecal samples show that MVT-201 administration causes a reduction in pathogen load 1000X times greater than the one observed in the untreated mice.

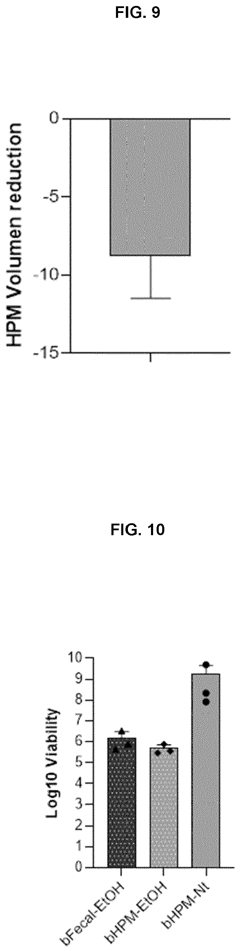

**Figure 9****.** Volume reduction compared to traditional FMT volume reduction of the IM obtained by the process of Example 1 when compared to homogenized whole fecal material used as starting material.
**Figure 10****.** Spore quantification in human purified microbiota. the number of viable spores present in the fecal homogenates (bFecal-EtOH) and in the human purified microbiota (bHPM-EtOH) compared to all bacteria present in the human purified microbiota sample (bHPM-Nt).

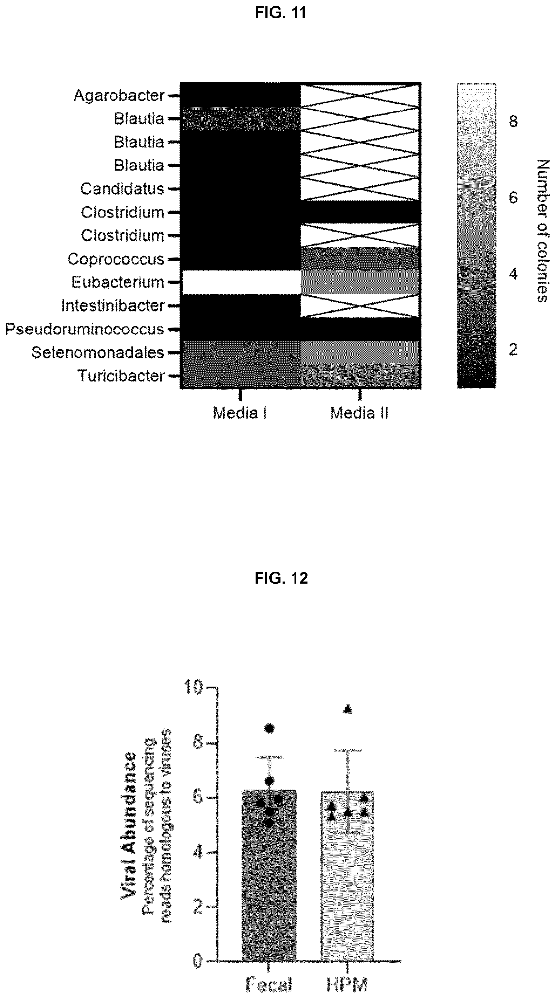

**Figure 11****.** Taxonomic classification of viable spores presents in purified microbiota. Heatmap showing the number of colonies from viable spores assigned to different species (only the genus is shown). The experiment was performed using two different growth media (Media I and Media II).
**Figure 12****.** Percentage of sequencing reads from purified microbiota metagenomes that are homologous to gut viruses. the number of sequencing-reads homologous to viral sequences in metagenomes obtained from fecal homogenates (Fecal; dark grey bar) and human purified microbiota (HPM; light grey bar).

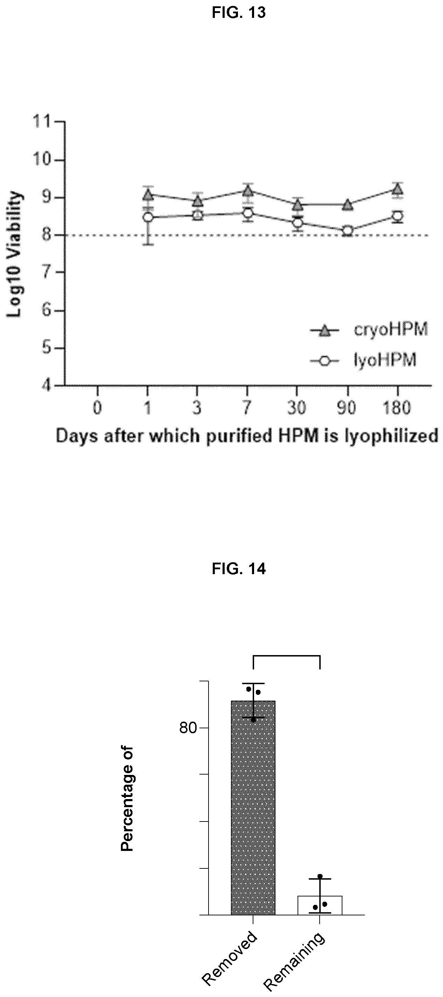

**Figure 13****.** Viability of purified microbiota that was freeze-dried (lyo-HPM) after different lengths of frozen storage (cryo-HPM) from the purification day. Viability measured in purified microbiota after it had been cryopreserved for different amount of time (x-axis) and subsequently lyophilized (lyoHPM; open circles) compared to viability measured in purified microbiota that had been cryoconserved for the same amount of time without being subjected to lyophilization process (cryoHPM; grey triangles).
**Figure 14****.** Percentage of spiked eukaryotic viral particles removed during microbiota purification. The percentage of eukaryotic viral genomes removed with the purification process (grey bar) and the percentage of spiked eukaryotic viral genomes remaining in the purified microbiota (white bar).

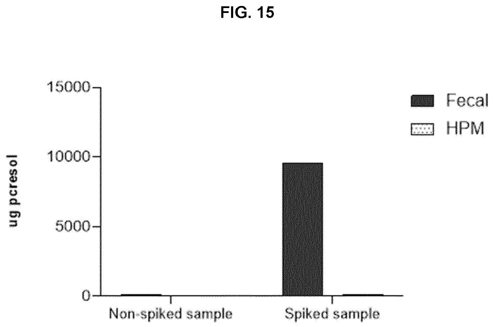

**Figure 15****.** Amount of p-cresol removed during microbiota purification. The amount of p-cresol detected in fecal samples and in purified microbiota is represented. The process was tested in the original sample (non-spiked) and in a sample that was spiked with a known amount of p-cresol (spiked sample). The purification process removes >99% of the p-cresol in the sample.

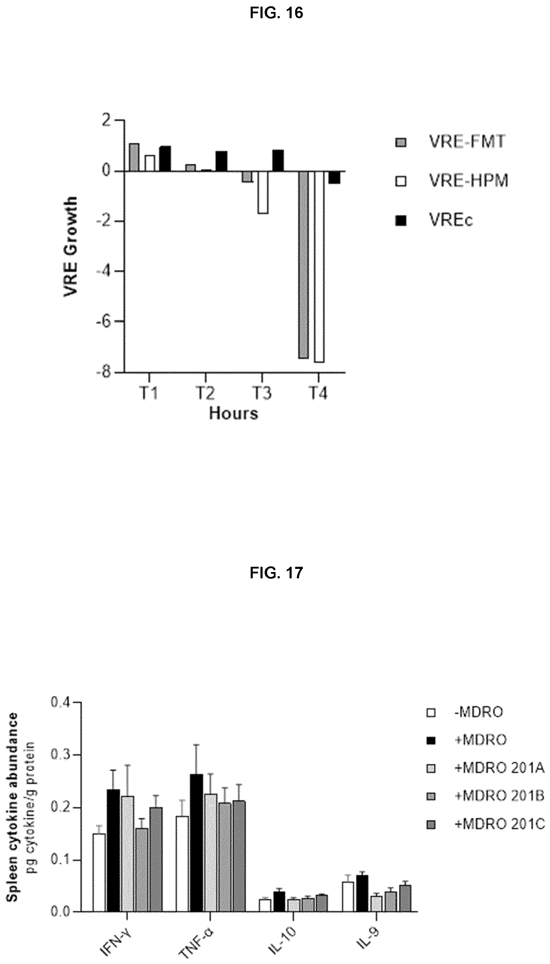

**Figure 16****.** Efficacy of fecal and purified microbiota in an inhibition in-vitro assay. VRE growth compared to initial values after incubation with fecal homogenate sample (VRE-FMT;grey bars), human purified microbiota (VRE-HPM; white bars) or buffer control (VREc; black bars). Negative values show inhibition of growth compared to initial values throughout time by the treatment
**Figure 17****:** Spleen cytokine profile after MVT201 treatment in a murine model of preclinal efficacy. Spleen cytokine levels in non-infected mice (-MDRO), infected mice (+MDRO), and infected mice treated with MVT201 (+MDRO 201A, B, C respectively) from three different donors are shown.

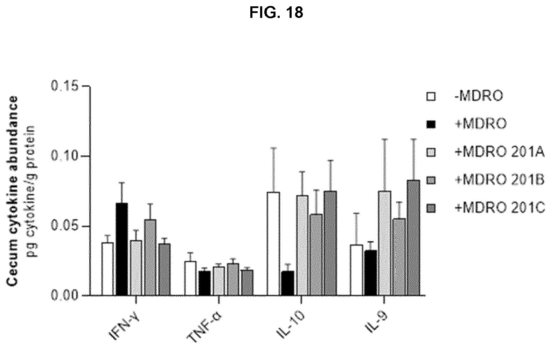

**Figure 18****:** Cecum cytokine profile after MVT201 treatment in a murine model of preclinal efficacy. Cecum cytokine levels in non-infected mice (-MDRO), infected mice (+MDRO), and infected mice treated with MVT201 (+MDRO 201A, B, C respectively) from three different donors are shown.

### DETAILED DESCRIPTION OF THE INVENTION

The present invention is directed to a method which allows isolation, purification, concentration and preservation of the intestinal microbiota (IM) from stool of a subject, and the therapeutical uses of the IM obtained.

In a first embodiment, the invention is directed to a process to obtain intestinal microbiota (IM) from a stool sample comprising the following steps:
a) homogenization of a fecal sample in saline solution with a reducing agent;
b) purification by density gradient;
c) washing with a reducing solution;
d) concentration; and
e) cryopreservation, dehydration, formulation and storage of the IM.

In a preferred embodiment, the saline solution in step a) is supplemented with 0.01 - 0.1% w/w, preferably 0.05% w/w of a reducing agent. Examples of reducing agents suitable for this step are sodium thioglycolate, iron salts, ascorbic acid and its salt (ascorbate), tocopherols (acid alpha-tocopherol), cysteine and its salt forms (hydrochloride cysteine in particular), vitamin K and mixtures of these forms. Preferably, the reducing agent is hydrochloride cysteine (Cys). The saline solution may be selected, for example, from NaCl, PBS or Ringer solution. Preferably, the saline solution is NaCl 0.9% w/v. For purposes of the present invention, the solution comprising the saline solution and the reducing agent is referred as reducing solution. In a more preferred embodiment, the homogenization occurs in anaerobiosis (in total or almost total absence of oxygen), for example in an anaerobic workstation.

The density gradient in step b) may be prepared of any compound suitable for this purpose. Preferably, the gradient is of Nycodenz (iohexol, 5-(N-2, 3-dihydroxypropylacetamido)-2, 4, 6-tri-iodo-N, N'-bis (2, 3 dihydroxypropyl) isophthalamide) 80%. In a preferred embodiment, the gradient is centrifuged at 5,000 - 15,000 xg, preferably 10,000 xg for 30 - 90 minutes, preferably 60 minutes at 2 - 8°C, preferably at 4°C.

The layer corresponding to the IM is extracted by any technique known in the art, and washed in step c), preferably twice, preferably with the same reducing solution as in step a). In a preferred embodiment, the sample is centrifuged at 10,000 - 15,000 xg, preferably at 12,000 xg for 5 - 15 minutes, preferably 10 minutes at 2 - 8°C, preferably at 4°C. More preferably, this step is performed in anaerobiosis (in total or almost total absence of oxygen), for example in an anaerobic workstation.

After the centrifugation, in step d) the supernatant is removed, and the IM is resuspended and concentrated in a saline solution comprising a cryo-thermoprotective agent. In a preferred embodiment, the cryo-thermoprotective agent is maltodextrin-trehalose. More preferably, this step is performed in anaerobiosis (in total or almost total absence of oxygen), for example in an anaerobic workstation.

In step e), the IM is cryopreserved, dehydrated, formulated and storage. The cryopreservation occurs between -196°C and -50°C, preferably at -80°C. The dehydration is by freeze-drying (lyophilized) or spray drying. Later, the dried product is formulated. For purposes of the present invention, a formulation is a mixture or a structure such as a capsule, tablet, or an emulsion, prepared according to a specific procedure. Formulations are a very important aspect of creating medicines, since they are essential to ensuring that the active part of the drug is delivered to the correct part of the body, in the right concentration, and at the right rate. In a preferred embodiment, the IM is encapsulated or formulated as a pill or a tablet. Finally, the formulated IM is stored between -80°C and room temperature, preferably between -80°C and 4°C.

In a preferred embodiment, the stool sample is from a healthy donor. A healthy donor is considered a person without any diagnosis disease, preferably without any disease related to the gastrointestinal tract. In other preferred embodiment, the stool sample is from a person with a diagnosed disease, preferably, but not exclusively, related to the gastrointestinal tract (i.e., autism).

The IM of the invention may be used for autologous administration. Autologous administration may be performed in a diseased subject, either with the IM obtained when the subject is diseased (stool sample from a diseased donor or modified IM), or with the IM obtained previously, when the subject was still healthy (healthy donor or healthy-own-IM). The aim of the addition of the IM from a diseased subject with their own IM is to increase, reduce or deplete bacterial species related with the disease or beneficial to the disease. Alternatively, the aim of the addition of the healthy-own IM is to reestablish a balanced microbial ecosystem associated with health.

The purified product obtained by the process disclosed herein mostly maintains the composition and diversity whereas the fecal components which may be a health risk for a diseased subject are completely or almost completely removed. These components are, for example, toxins, viruses, human cells and human DNA. **Figure 1** shows an overview of the process of the invention.

The process described herein maintains more than 95% of the bacterial taxa present in the original fecal sample and also maintains their relative abundance. This method renders an intestinal microbiota (IM) purified sample with a microbiological diversity comparable to the original sample. Obtaining a microbiota more similar to a naïve healthy microbiota with high diversity has been shown to work better when used in microbiota-based treatments (De Palma G et al., 2017. Sci Transl Med. 9(379):eaaf6397).

In a second embodiment, the invention is directed to the intestinal microbiota (IM) obtained from the process of the first embodiment described above.

In a preferred embodiment, the IM additionally comprises one or more microorganisms which do not belong to the donor, with the purpose of enriching the final product. The additional microorganism may be of the same species as one already present in the purified IM or of different species. In a more preferred embodiment, the microorganisms are selected from any species from the taxa Bacteroidetes, Firmicutes, Actinobacteria, Verrucomicrobia, Proteobacteria, or Fusobacteria. For example, but not limited to *Akkermansia, Faecalibacterium, Clostridium* cluster IV and XIVs, SCFA producers, *Christensenella,* etc. The additional one or more microorganisms are added before step e) or before the dried product is formulated.

The IM may be found in a dried form of powder, aerosol or liquid. Preferably, the IM is in the form of powder.

The IM may be formulated as tablets, capsules, pills, enema or suppositories with or without sustained release. In a preferred embodiment, the IM is formulated in a capsule.

In a third embodiment, the invention refers to a pharmaceutical composition comprising the intestinal microbiota (IM) of the second embodiment, and a pharmaceutically acceptable ingredient. Said additional pharmaceutical ingredient can be at least one excipient and/or at least one additional pharmaceutical active ingredient. Said additional pharmaceutical ingredient can be any adjuvant known in the state of the art as long as it does not affect, or it does not affect in an unacceptable manner the activity of the isolated IM of the present invention.

In a fourth embodiment, the invention is directed to the IM of the second embodiment of the pharmaceutical composition of the third embodiment as described above, for use as a medicament. In a preferred embodiment, the medicament is a biological drug. A biological drug is a drug comprising living organisms.

In a fifth embodiment, the invention is directed to the IM of the second embodiment or the pharmaceutical composition of the third embodiment as described above, for use in the treatment or prevention of a disease which courses with alteration of the IM or for use in the prevention of secondary or undesired effects after a medical treatment, or for use in reduction of the toxicity of a medical treatment in a subject. The undesired effects of a medical treatment are, for example but not limited to, gastrointestinal issues associated with antibiotic treatment and gastrointestinal or dermatological issues associated with oncological treatment. These include but are not limited to colitis, diarrhea or mucositis.

In a preferred embodiment, the disease is selected, without limitation, to an infectious disease, cancer, dermatological disease, autoimmune diseases, metabolic diseases, cardiovascular diseases, urogenital tract diseases, gastrointestinal disorders or neurodegenerative diseases. In a more preferred embodiment, the disease is selected from urinary tract infections (UTIs), cancer, metabolic or intestinal diseases and autoimmune diseases with or without antibiotic resistance bacteria. More preferably, the disease is cancer, and urinary or an intestinal infection, and even more preferably, the disease courses with antibiotic resistance bacteria.

In a sixth embodiment, the invention is directed to a probiotic or a nutraceutical composition comprising the IM of the second embodiment as described above.

The IM disclosed herein, has the following advantages over traditional processes:
i) lower risk of disease transmission;
ii) lower volume of product;
iii) easy administration without invasive processes. This means better adherence to a treatment;
iv) better stability of the product; and
v) reflection of the entire microbiota of a subject.

### EXAMPLES

### Example 1. Method to obtain IM from stool samples.

24 grams of a fecal sample from a healthy individual was introduced in a 400 mL stomacher bag and 100 mL of saline solution NaCl 0.9% supplemented with cysteine hydrochloride (Cys) 0.05% diluted 1:5 was added (reducing solution). The bag was introduced in a homogenized in a Stomacher homogenizer and incubated in an anaerobiosis cabinet.

Next, a density gradient was prepared by pipetting 10 mL of Nycodenz (iohexol, 5-(N-2, 3-dihydroxypropylacetamido)-2, 4, 6-tri-iodo-N, N'-bis (2, 3 dihydroxypropyl) isophthalamide) 80% in Falcon tubes. Subsequently, 30 mL of the homogenized sample were added, and the tubes were centrifuged with a Beckman JS-7.5 rotor in a high-speed swinging-rotor centrifuge at 10,000 xg for 60 minutes at 4°C.

The layer corresponding to the IM was extracted from the tubes under anaerobic conditions, and the IM was washed twice with 100 mL of NaCl 0.9% with Cys 0.05%. The sample was centrifuged at 12,000 xg for 10 minutes at 4°C in a fixed-angle Beckman centrifuge rotor JA-14.50.

The IM was concentrated at least twice in saline solution with the washing medium (reducing medium), and it was resuspended in saline solution with the cryo-thermoprotective agent Maltodextrin-trehalose under anaerobic conditions. The IM obtained was cryopreserved and dehydrated by freeze-drying or spray drying.

**Figure 1** shows the scheme of the process described in the Example 1.

### Example 2. Analysis of IM biodiversity.

It is desirable to have a bacterial composition in the IM as similar as possible, in terms of diversity, to the original microbiota of the healthy donor, in order to maintain the potentially beneficial ecosystem in the sample, without the fecal components which may be a health risk for the subject.

For this reason, IM obtained by the process of Example 1 was analyzed and compared with the maintenance of the bacterial taxa of IM obtained by a method or the prior art (Hevia A et al., 2015. Nature Scientific Reports 5: 16807, from now on, Hevia).

The procedure disclosed in Hevia analyzed the bacterial composition of the purified microbiota of eight individuals by 16S-rRNA gene sequencing. Partial 16S rRNA gene amplicons were obtained using V3-V4 region primers and sequenced using Ion Torrent PGM system. After sequencing, read groups were pre-processed with PGM software. These data were analyzed bioinformatically using QIIME 1.7.0. to determine the bacterial composition of the samples. This analysis demonstrated a significant reduction in the number of types of bacteria (alpha-diversity) since they lost an average of 60% of the taxonomic units (OTUs) identified during the purification process.

To determine whether the protocol of the present invention in the purification process improves the quality of the product, microbiota from nine individuals were purified and metagenomic analysis was carried out based on the 16S-rRNA gene sequence. The number of different operational taxonomic units in the original fecal sample and the purified IM was calculated and compared.

**Figure 2** shows the graphs comparing both protocols. Whereas Hevia loses 60% of the bacterial diversity, the protocol disclosed herein almost completely avoids the loss of OTUs, reducing the loss from 60% to 5%.

Principal Coordinate Analysis (PCoA) was performed. In this type of analysis, the overall microbial composition of the sample is represented in a 2D graph. Each point corresponds to a sample and the closer the points are, the more similar the bacterial composition is between those samples. Hevia showed that despite the loss in the number of OTUs, the global bacterial composition of the fecal samples (fecal- black points) was similar to the composition obtained after purification of the IM from the feces (purified- gray points), although the samples did not group well with their respective donors (**Figure 3A**). On the contrary, the IM obtained by the protocol of Example 1 (also known as MVT-201) is similar to the original microbiota (better grouping between fecal IM and purified IM within respective donors in PcoA), as shown in **Figure 3B**).

This comparative shows that the IM purification protocol of the invention almost completely eliminates the loss of alpha-diversity after the purification process. The taxonomic units found in the purified microbiota are reduced compared to those detected in homogenized feces (Hevia 2015, n=8, MVT-201 n=9). Only standard deviation of the samples of Figure 3B is shown, since individual points are not available in previously published data. As it may be seen, there is high diversity of IM maintained after purification.

The IM purification protocol established in the present document resulted in a purified IM more similar to the original donor's IM. This is an advantage because the aim is to preserve the composition as diverse and similar as possible to the original microbiota. This fact has been related to better effect in IM transplantation (De Palma G *et al.,* 2017).

### Example 3. Comparative analysis composition of the IM.

After having analyzed the diversity loss in the IM samples, the specific taxa affected by the purification process were analyzed as described in Hevia *et al.*

Hevia identified certain bacterial phyla and classes that were affected by the purification process (**Figure 4A****,** n=9). Specifically, an increase was identified in the Clostridia class, which represents more than 1% of the population of healthy individuals, and a reduction in less abundant classes such as Erysipelotrichi or several Proteobacteria was seen. The adaptations presented by the inventors manage to avoid the difference in abundances in these taxa (**Figure 4B****,** n=8).

The analysis at the phylum level demonstrates that the composition obtained with the process described herein is more similar to the original IM composition. The abundance of classes whose abundance is significantly affected during purification with the Hevia protocol, is not affected when using the protocol developed with the present invention.

Hevia also analyzed the bacterial viability of the purified microbiota of eight individuals by flow cytometry and demonstrated that viability was maintained relatively well after the purification process, always obtaining less than an order of magnitude loss of total viability. Similarly, the viability of the microbiota after purification with the protocol of Example 1 has been measured in eleven individuals by RT-qPCR, demonstrating that it contains on average more than 9 log units of viable bacteria and that the loss in viability does not exceed 0.5 orders of magnitude on average (see results in **Figure 5A** and **Figure 5B**).

To determine the effect of lyophilization on the bacterial composition in the final product (MVT-201), metagenomic analysis was carried out from fecal samples and lyophilized microbiota from previously analyzed individuals. DNA samples were sequenced using shotgun metagenomics and taxonomically assigned using MetaPhlan 3.0.7 with default parameters and CHOCOPhlan v201901 database. The IM purification protocol of the invention maintains high viability and a bacterial composition which reflects the donor's intestinal microbiota. **Figure 5C** shows the analysis of relative abundance of the different taxa in fecal microbiota (F) and microbiota purified (P) by the protocol of the Example 1 in various donors. The results show highly similar composition between fecal and purified samples, and a donor-dependent composition.

### Example 4. Stability of the purified microbiota over time

To determine the stability of the product over time, the microbiota of 23 healthy individuals was purified and viability was measured by plate counts at time 0, 3, 6, 12 and 24 months. In brief, microbiota was purified. Subsequently, serial dilutions were performed, plated in rich media plates and incubated in anaerobic conditions to count colony forming units per mL (CFUs/mL) of purified microbiota. The same was done with purified microbiota that was cryopreserved for different lengths of time up to 24 months.

As shown in **Figure 6****,** up to two years from freezing viability was not affected, but even so, it only represented an average loss of 0.71±0.58 log10 cfu/mL and a high number of viable bacterial cells continue to be maintained in the product.

### Example 5. Preclinical analysis of the purified microbiota

To evaluate the toxicity and intestinal colonization capacity of the purified IM (MVT-201), a preclinical study in a mouse model was conducted. The study lasted 42 days and 8-week-old C57BL/6 mice were used. The MVT-201 product was generated from 6 different donors- 3 men and 3 women. A control group received no treatment (Control), another group received antibiotic treatment for 12 days to eliminate the native microbiota (ATB-treated), and another group received antibiotics plus MVT-201 (single dose of 5x10⁹ CFU/mL; ATB+HPM). Within the MVT-201 group, six subgroups of mice were used, one per donor, with 5 mice per group (30 mice in total).

No significant differences were observed in the weight of the mice treated with antibiotics only nor in the group that received antibiotics together with MVT-201. There were also no differences in the weight of the organs between the groups, nor in the percentage of body weight that each organ represents. Therefore, it can be concluded that the drug MVT-201 has a good safety profile in murine models.

Pharmacodynamics of the product was evaluated within this preclinical study. The colonization dynamics of MVT201 in the recipients' intestine was determined using a murine model. The composition of the administered MVT-201 (labeled as Human Donor), and the fecal bacterial composition of the mice in the different groups was analyzed through 16S rRNA gene sequencing and bioinformatic analysis. **Figure 7A** shows that the administration of a single dose of MVT-201 generates a modulation of the fecal microbial composition of the mouse, this one being similar to the composition of the donor microbiota contained in the administered MVT-201. This modulation of the microbiota was observed in the two days after treatment after analyzing beta diversity (**Figure 7C**).

Furthermore, the taxonomic identification of the bacterial groups at the family level and the quantification of their relative abundance allowed to observe the similarity between the intestinal microbiota of the donor and that of the recipient group. At the species level, up to 23% of taxa were identical between the human donor microbiota and the murine fecal sample, after two days of MVT-201 administration (**Figure 7B**).

As expected, the presence of MVT-201 bacterial taxa in the murine intestine decreased over time, and after one week of MVT-201 administration, only 15% of taxa were identical in mouse and donor, 9%. After two weeks, 6% after three weeks and only 3% after four weeks. This decrease in the presence of MVT-201 microorganisms in the mouse is due to the fact that i) the administration of MVT-201 was carried out only once, ii) the native microbiota of the mouse and the human microbiota are very different, iii) that the physiological conditions of the mouse and human intestine are different, iv) when the administration of antibiotics is stopped and with the mouse's usual diet it tends to recover its native microbiota (unpublished results).

### Example 6. Effect of the product in intestine pathogen reduction

Finally, in order to determine the effectiveness of the isolated microbiota, a preclinical trial was carried out in a previously published murine model of intestinal colonization with MDR pathogens (Isaac et al., 2022. Nature Communications 13: 7718).

The study lasted 21 days and used 8-week-old C57BL/6 mice. The MVT-201 product was generated from 3 different donors. Five groups of mice were used, one per donor and two control groups (a total of 20 mice). All groups received antibiotic treatment (vancomycin) for seven days to eliminate the native microbiota and allow vancomycin-resistant Enterococcus (VRE) infection. Four days after starting treatment with vancomycin, four of the group received a VRE dose of 10⁶ CFU/mL. Three days after inoculation with VRE, vancomycin treatment was stopped, and the administration regimen began one day later (one donor per group). One of the groups only received VRE and did not receive the product MVT-201. In total, six doses of MVT-201 were administered per group interspersed over two weeks. **Figure 8A** shows the summary of the experiment.

Feces were collected over several days post-infection throughout the experiment to measure intestinal VRE levels by plating. MDR pathogen levels in fecal samples shows that MVT-201 administration causes a reduction in pathogen load 1000X times greater than the one observed in the untreated mice (**Figure 8B**).

In conclusion, the present invention has demonstrated the following:
- the adapted protocol almost completely eliminates the reduction in alpha-diversity;
- the adapted protocol avoids alterations in abundances of bacterial taxa;
- the product contains a purified IM similar to the original IM;
- the reduction in bacterial viability in product capsules is very low, and viability in capsules is high (values around 10⁹ cfu/mL);
- the frozen or freeze-dried product is stable over time;
- the product is safe in mice at a concentration of 5x10⁹ cfu/mL;
- the product accelerates the reduction of intestinal MDR pathogen in the first days of treatment and is capable of reducing intestinal colonization by more than two orders of magnitude compared to no treatment in a murine infection model.

### Example 7. Volume reduction compared to traditional FMT

It is desirable to have a product as concentrated as possible to reduce the intake of oral capsules and maximize adherence to treatment. The volume of homogenized fecal material, which is used in traditional fecal microbiota transplantation products (FMT), was compared to the volume of purified microbiota after the IM had been purified from homogenized fecal samples with the protocol of the invention (MV).

**Figure 9** shows the volume reduction of the IM obtained by the process of Example 1 when compared to homogenized whole fecal material. The microbiota obtained by the new process is 8 times more concentrated than the microbiota found in homogenized fecal material that is used in FMT.

### Example 8: Presence of bacterial spores and bacteriophages in Intestinal Microbiota present in MVT201

It is desirable to have a diverse IM with a composition similar to the donor's, including the presence of spores, which are likely to survive the passage through the intestinal tract.

Briefly, to quantify the number of viable spores present in fecal homogenates and in the human purified microbiota (HPM), samples were incubated with ethanol and washed. Subsequently, serial dilutions were performed, plated in rich media plates and incubated in anaerobic conditions to count colony forming units per mL (CFUs/mL) of spore forming bacteria. The same was done with samples not treated with ethanol to quantify the original number of spores present in the sample. **Figure 10** shows the number of spores present in the fecal homogenates (bFecal-EtOH) and the human purified microbiota (bHPM-EtOH) compared to all bacteria present in the human purified microbiota sample (bHPM-Nt). The results indicate the purification process does not affect significantly the number of viable spores present in the IM found in MVT201 (average of 6.02 vs 5.63 log10 CFUs/ml respectively). This result was confirmed in several media (data not shown).

The taxonomic classification of the spore forming bacteria that were isolated in different media was determined (**Figure 11**). For this purpose, DNA was isolated from colonies grown in two types of media (Media I- modified BHI and Media II- RCM), and the 16S ribosomal RNA gene was sequenced. The results demonstrated that the spore forming bacteria belong to a wide range of bacterial taxa, including commonly known spore forming genera such as *Clostridium.*

The data demonstrated that the process of the invention avoids the loss of important bacterial taxa and that the composition present in the obtained IM is similar to the donor's IM. In the present experiment, it was examined the presence of bacterial spores and bacteriophages in the obtained IM.

Bacteriophages are an important factor regulating the ecology of bacterial communities. More than 98% of gut viruses are bacteriophages (also known as phages), and most of these are inserted in the genome of their bacterial host (known as prophages). It was presumed that, despite the purification process, most prophages are transferred inside the purified bacteria present in the IM obtained by the process of the invention. To confirm this hypothesis, metagenomic analysis was carried out.

Briefly, DNA from fecal homogenates and purified intestinal microbiota present in MVT201 was extracted and sequenced using shotgun metagenomics. To identify sequencing reads that belong to intestinal bacteriophages and viruses, reads were taxonomically classified using a Gut Virome specific database, which is composed primarily of gut bacteriophage sequences (Gregory AC et al., 2020. The Gut Virome Database Reveals Age-Dependent Patterns of Virome Diversity in the Human Gut. Cell Host & Microbe 28 (5); 724-40). As shown in **Figure 12****,** the number of sequencing-reads homologous to viral sequences in the metagenomes obtained from purified microbiota are comparable to those from fecal samples.

### Example 9. Stability of the purified microbiota over time

Previously, the stability of purified microbiota over time had been determined. This study concluded that purified microbiota was stable for up to two years. This study has been expanded, in order to determine the viability of purified microbiota lyophilized after different lengths of frozen storage.

Briefly, frozen microbiota obtained by the process of Example 1 that had been stored for different lengths of time (1, 3, 6 and 12 months) was lyophilized, after which the viability was measured by plate counts. Subsequently, serial dilutions were performed, plated in rich media plates and incubated in anaerobic conditions to count colony forming units per mL (CFUs/mL) of lyophilized purified microbiota (lyoHPM in **Figure 13**). As a control, it was used purified microbiota cryopreserved for the same amount of days, but not lyophilized (cryoHPM). As shown in the figure, the viability of powdered MVT201 was not significantly different, regardless of the length of time that it had been cryopreserved before lyophilization, In conclusion, purified microbiota is stable overtime and a high number of viable bacterial cells continue to be maintained in the product after cold storage and lyophilization.

### Example 10. Toxicity and safety profile of purified microbiota

Previously, the toxicity of MVT201 had been tested during preclinical studies using murine models. Overall, this study demonstrated that MVT201 was safe in mice. Because MVT201 is a donor derived product, the inventors wanted to expand its safety profile. One of the potential risks of using donor-based microbiome therapies is the transmission of unknown viruses that cannot be tested for during the extensive security screening that the donor samples are subjected to.

To determine whether the protocol of the invention can effectively reduce the presence of eukaryotic viral particles, the amount of virus that was removed after obtaining the IM was quantified. Briefly, a known amount of inactivated SARS-Cov2 virus was spiked into a fecal sample before purification. Samples were taken before the spike and throughout the purification process. Subsequently, the genetic material was extracted, and the amount of viral nucleic acid was quantified using quantitative RT-PCR with primers specific to the virus. This experiment was performed with three independent fecal samples. As shown in **Figure 14****,** the protocol of the invention is able to eliminate more than 90% of the eukaryotic viral particles in the fecal sample. This result demonstrates that MVT201 is safer than homogenized whole fecal material.

An additional advantage of the process is the elimination of potentially harmful metabolites that can be associated with the donor's diet. One example is the metabolite p-cresol, which is produced after protein fermentation and can induce DNA damage, potentially harming colonocytes (Zhang W et al., 2021. Gut Microbiota-Derived Metabolites in Colorectal Cancer: The Bad and the Challenges. Front. Oncol. Sec. Gastrointestinal Cancers: Colorectal Cancer 11, Article 739648). To test whether the protocol could effectively reduce the amount of metabolites found in feces, the amount of p-cresol that was removed during the purification process was quantified. Because p-cresol is found in fecal samples at different concentrations, each fecal sample was divided into two parts prior to purification. One of them was spiked with a known amount of p-cresol above the normal physiological range (spiked sample). The microbiota from both samples was purified. Throughout the process, aliquots were processed to quantify the amount of p-cresol in each step. As shown in **Figure 15** p-cresol is found in the stool sample within the physiological range and this metabolite is eliminated completely after purification. A similar result is obtained for the spiked sample, where the process is able to eliminate >99% of the p-cresol that was spiked into the sample.

This result provides further evidence that the purification of microbiota results in a potentially safer product compared to conventional fecal microbial transplantation.

### Example 11. Efficacy of MVT201 compared to whole fecal sample

Conventionally, whole stool sample has been used for fecal microbial transplantation. To determine whether the use of purified microbiota is as effective as the use of the whole fecal sample, an *in vitro* vancomycin resistant Enterococcus (VRE) inhibition was performed.

For this assay, a VRE previously used to test the efficacy of VMT201 *in vivo* grown in the laboratory. In parallel, microbiota was purified from a fecal sample. An aliquot of the fecal sample and of the purified microbiota were cryopreserved. Subsequently, purified microbiota or fecal sample were mixed with VRE cultures and incubated at 37°C for several days. As shown in **Figure 16****,** the results from this assay demonstrate that purified microbiota can be as effective as the homogenized fecal sample.

### Example 12. MVT201 regulation of the immune system

Regulation of the immune system is one of the mechanisms of action that have been proposed during the treatment of intestinal infections with intestinal microbiota transplantation. To test whether MVT201 produced in Example 1 is able to regulate local and systemic immunity we analyzed the changes in cytokine production after administration of MVT201 in the murine model of intestinal infection used in Example 6. Cytokines from spleen (systemic immunity) and cecum (local immunity) were isolated and profiled using Flow Cytometry.

**Figure 17** shows that the levels of cytokines related to inflammation, such as TNF-α and IFN-γ, were increased in spleen after VRE infection (+MDRO) compared to the non-infected groups (-MDRO). In contrast, the mice that had received MVT201 (+MDRO 201A, + MDRO 201C + MDRO 201C) had lower levels of these cytokines, more similar to the levels detected in the non-infected group. These changes were more pronounced in MVT201 from certain donors.

**Figure 18** shows that IFN-γ levels were also increased in the cecum of infected mice compared to uninfected mice. As seen in the spleen, the levels in the MVT201 mice were lower and similar to the non-infected group. The levels of cytokines involved in immune homeostasis, such as IL10 and IL9 were also affected. IL10 levels were greatly reduced in infected mice (+VRE). However, mice that received MVT201 (+MDRO 201A, +MDRO 201C +MDRO 201C) had increased levels similar to non-infected mice (-MDRO). Lastly, IL9 was greatly increased in mice that received MVT201 compared to both non-infected and infected mice.

Overall, the data provided demonstrates that:
- The method of the invention significantly reduces the volume in which the intestinal microbiota is resuspended,
- The process maintains the amount of bacterial spores
- The process maintains bacterial viruses that are important for microbiota ecology
- The frozen product can be stored and lyophilized without viability loss for up to 12 months (maximum tested)
- The process reduces the amount of eukaryotic viruses that could be potentially harmful.
- The process reduces the amount of potential harmful metabolites present in the fecal homogenate.
- The purified microbiota reduces the viability of VRE in vitro at levels comparable or better than whole microbiota treatment.
- The microbiota present in MVT201 is able to regulate the immune system in a murine model of intestinal microbiota.

## Claims

1. A process to obtain intestinal microbiota (IM) from a stool sample comprising the following steps:
a) homogenization of the sample in a saline solution with 0.01 - 0.1% w/w of a reducing agent;
b) purification by density gradient;
c) washing with the reducing solution of step a);
d) concentration; and
e) cryopreservation between -196 °C and -50 °C, dehydration, formulation and storage of the IM.

2. The process of claim 1, wherein the reducing agent is selected from the group consisting of sodium thioglycolate, iron salts, ascorbic acid and its salt (ascorbate), tocopherols (acid alpha-tocopherol), cysteine and its salt forms (hydrochloride cysteine), vitamin K and mixtures of these forms.

3. The process of claim 4, wherein the reducing agent is hydrochloride cysteine (Cys).

4. The process of any of claims 1 to 3, wherein the saline solution is selected from the group consisting of NaCl, PBS or Ringer solution.

5. The process of any of claims 1 to 4, wherein steps a), b), c) and d) occur in anaerobiosis.

6. The process of any of claims 1 to 5, wherein the density gradient in step b) is of Nycodenz (iohexol, 5-(N-2, 3-dihydroxypropylacetamido)-2, 4, 6-tri-iodo-N, N'-bis (2, 3 dihydroxypropyl) isophthalamide) 80%.

7. The process of any of claims 1 to 6, wherein the cryo-thermoprotective agent is maltodextrin-trehalose.

8. The process of any of claims 1 to 7, wherein the stool sample is from a healthy donor.

9. The intestinal microbiota (IM) obtained from the process of any of claims 1 to 8.

10. The IM of claim 9, wherein the IM additionally comprises one or more microorganisms which do not belong to the donor of the stool sample.

11. The IM of claim 10, wherein the additional one or more microorganisms are selected from any species from the phyla Bacteroidetes, Firmicutes, Actinobacteria, Verrucomicrobia, Proteobacteria, Fusobacteria.

12. The IM of any of claims 9 to 11, wherein the IM is formulated as tablets, capsules, pills, enema or suppositories with or without sustained release.

13. A pharmaceutical composition comprising the intestinal microbiota (IM) of any of claims 9 to 12 and a pharmaceutically acceptable ingredient.

14. The IM of any of claims 9 to 12 or the pharmaceutical composition of claim 13, for use as a medicament.

15. The IM of any of claims 9 to 12 or the pharmaceutical composition of claim 13, for use in the treatment of a disease selected from an infectious disease, cancer, a dermatological disease, autoimmune diseases, metabolic diseases, cardiovascular diseases, urogenital tract diseases, gastrointestinal disorders, neurodegenerative diseases, gastrointestinal issues associated with antibiotic treatment, or gastrointestinal or dermatological issues associated with oncological treatment.

16. The IM for use according to claim 15, wherein the disease is selected from an infectious disease with antibiotic resistance pathogen, a urinary tract infection (UTI), cancer, a gastrointestinal disorder, a metabolic disease or an autoimmune disease.

17. The IM of any of claims 9 to 12 or the pharmaceutical composition of claim 13 for use as adjuvant.

## Patentansprüche

1. Verfahren zum Gewinnen der intestinalen Mikrobiota (IM) aus einer Stuhlprobe, umfassend die folgenden Schritte:
a) Homogenisieren der Probe in einer Kochsalzlösung mit 0,01-0,1 % w/w eines Reduktionsmittels;
b) Reinigen mittels Dichtegradienten;
c) Waschen mit der Reduktionslösung aus Schritt a);
d) Konzentrieren; und
e) Kryokonservieren zwischen -196 °C und -50 °C, Dehydrieren, Formulieren und Lagern der IM.

2. Verfahren nach Anspruch 1, wobei das Reduktionsmittel aus der Gruppe ausgewählt ist, die aus Natriumthioglykolat, Eisensalzen, Ascorbinsäure und deren Salzen (Ascorbat), Tocopherolen (alpha-Tocopherolsäure), Cystein und dessen Salzformen (Cysteinhydrochlorid), Vitamin K sowie Gemischen dieser Formen besteht.

3. Verfahren nach Anspruch 4, wobei das Reduktionsmittel Cysteinhydrochlorid (Cys) ist.

4. Verfahren nach einem der Ansprüche 1 bis 3, wobei die Kochsalzlösung aus der Gruppe ausgewählt ist, die aus NaCl, PBS oder Ringer-Lösung besteht.

5. Verfahren nach einem der Ansprüche 1 bis 4, wobei die Schritte a), b), c) und d) unter anaeroben Bedingungen ablaufen.

6. Verfahren nach einem der Ansprüche 1 bis 5, wobei der Dichtegradient in Schritt b) aus Nycodenz (Iohexol, 5-(N-2,3-Dihydroxypropylacetamido)-2,4,6-triiodo-N,N'-bis(2,3-dihydroxypropyl)isophthalamid) zu 80 % besteht.

7. Verfahren nach einem der Ansprüche 1 bis 6, wobei das kryothermoprotektive Mittel Maltodextrin-Trehalose ist.

8. Verfahren nach einem der Ansprüche 1 bis 7, wobei die Stuhlprobe von einem gesunden Spender stammt.

9. Intestinale Mikrobiota (IM), die durch das Verfahren nach einem der Ansprüche 1 bis 8 gewonnen wurde.

10. IM nach Anspruch 9, wobei die IM zusätzlich einen oder mehrere Mikroorganismen umfasst, die nicht zum Spender der Stuhlprobe gehören.

11. IM nach Anspruch 10, wobei das eine oder die mehreren zusätzlichen Mikroorganismen aus beliebigen Arten der Stämme Bacteroidetes, Firmicutes, Actinobacteria, Verrucomicrobia, Proteobacteria und Fusobacteria ausgewählt sind.

12. IM nach einem der Ansprüche 9 bis 11, wobei die IM in Form von Tabletten, Kapseln, Pillen, Einläufen oder Zäpfchen mit oder ohne verzögerte Freisetzung vorliegt.

13. Pharmazeutische Zusammensetzung, umfassend die intestinale Mikrobiota (IM) nach einem der Ansprüche 9 bis 12 und einen pharmazeutisch akzeptablen Inhaltsstoff.

14. IM nach einem der Ansprüche 9 bis 12 oder pharmazeutische Zusammensetzung nach Anspruch 13, zur Verwendung als Arzneimittel.

15. IM nach einem der Ansprüche 9 bis 12 oder pharmazeutische Zusammensetzung nach Anspruch 13, zur Verwendung bei der Behandlung einer Krankheit, ausgewählt aus einer Infektionskrankheit, Krebs, einer Hautkrankheit, Autoimmunerkrankungen, Stoffwechselerkrankungen, Herz-Kreislauf-Erkrankungen, Erkrankungen des Urogenitaltrakts, Magen-DarmErkrankungen, neurodegenerativen Erkrankungen, Magen-Darm-Beschwerden, die mit einer Antibiotikatherapie verknüpft sind, oder Magen-Darm- bzw. Hautbeschwerden, die mit einer onkologischen Behandlung verknüpft sind.

16. IM zur Verwendung nach Anspruch 15, wobei die Krankheit aus einer Infektionskrankheit mit einem antibiotikaresistenten Erreger, einer Harnwegsinfektion (UTI), Krebs, einer Magen-Darm-Erkrankung, einer Stoffwechselerkrankung oder einer Autoimmunerkrankung ausgewählt ist.

17. IM nach einem der Ansprüche 9 bis 12 oder pharmazeutische Zusammensetzung nach Anspruch 13 zur Verwendung als Adjuvans.

## Revendications

1. Procédé d'obtention d'un microbiote intestinal (IM) à partir d'un échantillon de selles, comprenant les étapes suivantes :
a) homogénéisation de l'échantillon dans une solution saline contenant de 0,01 à 0,1 % p/p d'un agent réducteur ;
b) purification par gradient de densité ;
c) lavage avec la solution réductrice de l'étape a) ;
d) concentration ; et
e) cryoconservation entre -196 °C et -50 °C, déshydratation, formulation et stockage de l'IM.

2. Procédé selon la revendication 1, dans lequel l'agent réducteur est choisi dans le groupe constitué de thioglycolate de sodium, de sels de fer, d'acide ascorbique et de son sel (ascorbate), de tocophérols (acide alpha-tocophérol), de cystéine et de ses formes salines (chlorhydrate de cystéine), de vitamine K et de mélanges de ces formes.

3. Procédé selon la revendication 4, dans lequel l'agent réducteur est le chlorhydrate de cystéine (Cys).

4. Procédé selon l'une quelconque des revendications 1 à 3, dans lequel la solution saline est choisie dans le groupe constitué de NaCl, de PBS ou de solution de Ringer.

5. Procédé selon l'une quelconque des revendications 1 à 4, dans lequel les étapes a), b), c) et d) ont lieu en anaérobiose.

6. Procédé selon l'une quelconque des revendications 1 à 5, dans lequel le gradient de densité de l'étape b) est constitué de Nycodenz (iohexol, 5-(N-2,3-dihydroxypropylacétamido)-2,4,6-tri-iodo-N,N'-bis(2,3-dihydroxypropyl)isophtalamide) à 80 %.

7. Procédé selon l'une quelconque des revendications 1 à 6, dans lequel l'agent cryothermoprotecteur est la maltodextrine-tréhalose.

8. Procédé selon l'une quelconque des revendications 1 à 7, dans lequel l'échantillon de selles provient d'un donneur sain.

9. Microbiote intestinal (IM) obtenu par le procédé selon l'une quelconque des revendications 1 à 8.

10. IM selon la revendication 9, dans lequel l'IM comprend en outre un ou plusieurs micro-organismes n'appartenant pas au donneur de l'échantillon de selles.

11. IM selon la revendication 10, dans lequel lesdits un ou plusieurs micro-organismes supplémentaires sont choisis parmi toute espèce des phylums Bacteroidetes, Firmicutes, Actinobacteria, Verrucomicrobia, Proteobacteria et Fusobacteria.

12. IM selon l'une quelconque des revendications 9 à 11, dans lequel l'IM est formulé sous forme de comprimés, de capsules, de pilules, de lavement ou de suppositoires, avec ou sans libération prolongée.

13. Composition pharmaceutique comprenant le microbiote intestinal (IM) selon l'une quelconque des revendications 9 à 12 et un ingrédient pharmaceutiquement acceptable.

14. IM selon l'une quelconque des revendications 9 à 12 ou composition pharmaceutique selon la revendication 13, pour son utilisation en tant que médicament.

15. IM selon l'une quelconque des revendications 9 à 12 ou composition pharmaceutique selon la revendication 13, pour son utilisation dans le traitement d'une maladie choisie parmi une maladie infectieuse, un cancer, une maladie dermatologique, des maladies auto-immunes, des maladies métaboliques, des maladies cardiovasculaires, des maladies des voies urogénitales, des troubles gastro-intestinaux, des maladies neurodégénératives, des problèmes gastro-intestinaux associés à un traitement antibiotique, ou des problèmes gastro-intestinaux ou dermatologiques associés à un traitement oncologique.

16. IM pour son utilisation selon la revendication 15, dans lequel la maladie est choisie parmi une maladie infectieuse due à un agent pathogène résistant aux antibiotiques, une infection des voies urinaires (IVU), un cancer, un trouble gastro-intestinal, une maladie métabolique ou une maladie auto-immune.

17. IM selon l'une quelconque des revendications 9 à 12 ou composition pharmaceutique selon la revendication 13, pour son utilisation en tant qu'adjuvant.
